# EUROPEAN PATENT SPECIFICATION

(11) **EP 1 696 967 B1**
(45) Date of publication and mention of the grant of the patent: **22.05.2013**
(21) Application number: 04815384.5
(22) Date of filing: 22.12.2004
(51) Int. Cl.: A61L 2/02, D06M 15/61, D06M 15/55, D06M 23/08, D06M 11/45, D06M 11/79, D01F 1/10

(54) **BACTERIA BINDING PRODUCTS**
BAKTERIEN-BINDUNGSPRODUKTE
PRODUITS DE FIXATION DES BACTERIES

(30) Priority: 23.12.2003 US 745266
(43) Date of publication of application: 06.09.2006
(73) Proprietor: KIMBERLY-CLARK WORLDWIDE, INC., Neenah, WI 54956 (US)
(72) Inventor: VILLANUEVA, Julie, M., Decatur, Georgia 30030 (US); SAYRE, Curtis, Neil, Atlanta, Georgia 30306 (US); HUANG, Lei, Duluth, Georgia 30097 (US); MCGRATH, Kevin, Peter, Alpharetta, Georgia 30004 (US); WEI, Ning, Roswell, Georgia 30075 (US)
(74) Representative: Leidescher, Thomas
(86) International application number: PCT/US2004/043301
(87) International publication number: WO 2005/063307

(56) References cited:
- WO-A-00/36207
- WO-A-01/83665
- WO-A-2004/004679
- WO-A2-2004/038089
- DATABASE WPI Section Ch, Week 200471 Derwent Publications Ltd., London, GB; Class F04, AN 2001-138872 XP002326334 & CN 1 057 807 C (SHU J) 25 October 2000 (2000-10-25)
- DATABASE WPI Section Ch, Week 199340 Derwent Publications Ltd., London, GB; Class A85, AN 1993-315088 XP002326335 & JP 05 226187 A (TORAY IND INC) 3 September 1993 (1993-09-03)
- DATABASE WPI Section Ch, Week 199236 Derwent Publications Ltd., London, GB; Class A88, AN 1992-294870 XP002327445 & JP 04 197409 A (UNITIKA RES LAB KK) 17 July 1992 (1992-07-17)

## Description

### BACKGROUND OF THE INVENTION

The invention concerns wipes for the binding and removal of negatively charged particles like bacteria and other microbes without the use of harsh chemicals.

As concern grows about allergic reactions to chemicals and about the increasing resistance of bacteria to common drug treatments, so has the concern and desire for products that avoid harsh chemicals yet still achieve their purpose. In the case of currently available wet wipes, for example, the wipe is impregnated with a solution of chemicals. A typical chemical may be an antimicrobial chemical and the use of the wipe helps deliver the chemicals to the contaminated surfaces. More desirably, however, a wipe would retain the chemicals while removing the germs from the surface. A wipe that removes the bacteria but which does not leave chemicals on the surface provides the desired decontamination effect without the undesirable exposure of people to the chemicals.

A myriad of different consumer products may benefit from this type of bacterial removal. It is an object of this invention is to provide products that remove negatively charged particles without leaving a residue of chemicals.

### SUMMARY OF THE INVENTION

In response to the foregoing difficulties encountered by those of skill in the art, we have developed wipes for the binding and removal of negatively charged particles like bacteria from surfaces. The wipes have a positive charge that is developed through the use of cationic treatments. The treatment chemicals are selected from positively charged nanoparticles and an alumina oligomer. After the treatment is applied, the resulting product may be treated with heat at a temperature and for a time sufficient to crosslink the coating and attach the coating to the substrate.

The treatments suitable for use herein do not oxidize the surface of the product to which they are applied. This avoids the need for very harsh conditions during product manufacturing. The treatments are, however, cross-linked with the surface of the product.

### BRIEF DESCRIPTION OF THE DRAWINGS

Figure 1 is a drawing of a diaper.
Figure 2 is a drawing of a training pant.
Figure 3 is a drawing of a feminine hygiene pad.
Figure 4 is a drawing of an absorbent underpant.

### DETAILED DESCRIPTION OF THE INVENTION

The present invention involves the binding and removal of negatively charged particles like bacteria, cells, allergens, pathogens and molecules from surfaces. This has become increasingly important to consumers as the number of bacteria resistant to common treatments has risen. It is also increasingly important to consumers that they not be exposed to harsh chemicals.

Products incorporating the chemistry herein remove negatively charged particles from surfaces. The negatively charged particles are removed without the use of harsh chemicals, i.e., chemicals that are caustic or cause irritation to the skin of the average person, so the consumer is not required to touch a chemical-laden item. In the case of bacteria, for example, since the bacteria are not exposed to chemicals that will kill them in substantial numbers, i.e., more than 20 percent, the bacteria are not prompted to develop immunities to the chemical treatment on the product substrates. The bacteria are simply removed through the application of physical means and Coulombic attraction. A successful treatment compound does not leach from a substrate in a sufficient quantity to substantially kill bacteria and will therefore have at least 80 percent survival of a control colony, i.e., the treatment will kill 20 percent or less of the bacterial colonies.

Wipes are contemplated for the removal of negatively charged particles as taught herein.

Nonwoven fabrics are generally bonded in some manner as they are produced in order to give them sufficient structural integrity to withstand the rigors of further processing into a finished product. Bonding can be accomplished in a number of ways such as hydroentanglement, needling, ultrasonic bonding, adhesive bonding, stitchbonding, through-air bonding and thermal bonding, all of which are suitable for the practice of this invention.

The treatment has a positive charge in order to attract and hold the negatively charged particles. Positive charges may be generated in a number of ways; a cationically charged chemical treatment is added to the product, and an electret treatment may be applied to the product, resulting in a positive charge. Suitable chemicals include positively charged nanoparticles and alumina oligomers.

Examples of suitable alumina oligomers are aluminum chlorohydrol and aluminum chlorohydrate.

Nanoparticles like SNOWTEX® AK from Nissan Chemicals Inc., of Houston, TX, USA and aluminum chlorohydrate from Reheis, Inc. of Berkeley Heights, NJ, USA, may be used. In addition to having a positive charge, the chemicals suitable for the practice of the invention are mild in their effect on the skin, not appreciably antimicrobial in nature and do not leach substantially once bonded to the surface of the substrate.

The amount of chemical that should be added will vary according to the amount of charge the particular chemical chosen will contribute. Generally however, the effective amount of chemical will be between about 0.01 and 10 weight percent, more desirably between 0.05 and 7 weight percent, and still more desirably between 0.1 and 5 weight percent.

The chemical treatment may be applied by methods such as traditional dip and squeeze techniques, where the item is dipped into the chemical treatment and excess chemical is squeezed off, or by brush coating, spraying, ink-jet printing, and the like. It is also possible to add the chemical treatment as an internal treatment to, for example, a polymer fiber, as discussed below.

The chemically treated product surface may be treated with heat at a temperature and for a time sufficient to crosslink the coating and adhere it to the web. In the case of alumina oligomers, the crosslinking process involves Al-OH groups of the oligomer and OH from either the oligomer (intramolecular crosslinking) or OH group from the substrate (intermolecular crosslinking). It's believed that the nanoparticles coated with alumina oligomer would adhere to OH-containing surfaces by crosslinking the OH group with Al-OH groups of the oligomer. The combination of time and temperature sufficient to crosslink the polymer will depend on the polymer and substrate chosen. Generally speaking however, the time will be between 1 and 60 minutes, more desirably between 5 and 45 minutes, still more desirably between 15 and 35 minutes, with a temperature between about 50 and 300 °C, more desirably between about 80 and 200 °C, still more desirably between about 90 and 125 °C. The inventors have found, for example, that a temperature of 100 °C for about 20 to 30 minutes cures many of the polymers of interest.

Depending on the nature of the fibers, functionlized polymers (such as KYMENEs® with epoxy groups) are capable of involving both intra-molecular (i.e., only within the coating layer) and inter-molecular (i.e., only with the substrate) crosslinking processes. It's believed to be likely that the crosslinking process will combine both intra-molecular and intermolecular processes if the substrates are functionalized. Alternatively, if the substrate is not capable of participating in the chemical crosslinking process, then only intramolecular crosslinking may occur. In either case, a durable coating is often obtained when the non-functionalized substrate is made wettable by pre-treating before coating. The term "adhere to the substrate" includes, therefore, instances of intramolecular crosslinking that create a "sleeve" around the fibrous substrate, as well as intermolecular crosslinking where the chemical or a carrier of the chemical (such as a nanoparticle coated with an alumina oligomer) forms a covalent bond on the substrate, and combinations thereof. A cationically charged chemical "adheres to the substrate" if it does not leach from the substrate during use, where "not leach" from a substrate means that the concentration of the chemical in the liquid left on a surface with which the substrate comes into contact, is less than the critical concentration for the chemical to have antimicrobial properties.

Alternatively, the cationically charged compound may be imbedded in a product made from fibers by melt-extruding the fiber-forming polymer containing a desired amount of the cationically charged compound as an additive in the fibers of the web. Such compounds may "bloom" to the surface when the web is exposed to hydrophilic solvents such as water. These melt extrudable fibers may contain a polyolefin and a cationically charged compound. The cationically charged compound may also contain a chemical segment (i.e., compatibilizer) that is soluble in the polyolefin such that the salt is compatibilized with the polymer. The cationically charged chemicals may be, for example, amphiphilic quaternium ammonium salts that are compatible with hydrophobic webs, examples of which are taught by Nohr and Macdonald in US patent 5,853,883.
If the hydrophobic segment of the salt that is compatible with the hydrophobic polymer is relatively large (with respect to the ionic segment of the salt) such that the amount of salt that leaches out of the web is insufficient to kill bacteria, then the web would not have antimicrobial activity. The cationically charged groups generally come to the surface of the predominately polymeric fibers when the web is exposed to water. Such blooming gives the webs properties similar to those of substrates coated with cationically charged compounds.

The inventors tested numerous substrates and chemical treatments for their removal efficiency and chemical leaching. These materials, treatments, test procedures and results are shown below.

PBS Control: This refers to sterile phosphate buffered saline (PBS) and indicates that no fabric, treatment or negatively charged particles were present in this sample. Phosphate buffered saline (available from Gibco and Invitrogen at 10X concentration) is diluted to 1X with distilled water and sterile filtered before using.

Spunlace fabric: The spunlace process is also known as hydroentanglement. The spunlace process subjects the fiber web to fine jets of water at high pressures. When the jets strikes the web, it repositions and entangles the fibers into an interlocked "spunlace" web. The web is then dried in hot ovens. Generally speaking, spunlace webs contain no chemical binders, and they have an excellent textile-like drape and softness, good mechanical and aesthetic properties, and good absorbency and wetting. A wide range of natural and synthetic fibers can be used to make spunlace webs, including polypropylene, rayon, PET, and nylon. Staple fibers are also used in spunlace nonwovens products. The spunlace fabric tested herein was made from 65 weight percent rayon and 35 weight percent PET. The fabric was tested in the uncreped state as well as in the creped state where the creping was carried out in accordance with US patents 6,197,404 and 6,150,002.

These creped materials have regions of interfilament bonding which are permanently bent out-of-plane, alternating with regions of no interfilament bonding. The non-bonded regions include a multiplicity of filament loops which terminate at bond ends in the creped interfilament bonded regions.

Fuzzy film, polyurethane: This fuzzy film is made of polyurethane (PU) foam and polyethylene (PE) film through a tack-spinning process. In tack-spinning, the PU and PE are laminated together, the PE is partially melted on a hot roller and the surface is fiberized by pulling the material away from the roller and blowing air on/through it to cool it:

Bonded Carded web: "Bonded carded web" refers to webs which are made from staple fibers which are sent through a combing or carding unit, which breaks apart and aligns the staple fibers in the machine direction to form a generally machine direction-oriented fibrous nonwoven web. Such fibers are usually purchased in bales which are placed in a picker which separates the fibers prior to the carding unit. Once the web is formed, it then is bonded by one or more of several known bonding methods. One such bonding method is powder bonding, wherein a powdered adhesive is distributed through the web and then activated, usually by heating the web and adhesive with hot air. Another suitable bonding method is pattern bonding, wherein heated calender rolls or ultrasonic bonding equipment are used to bond the fibers together, usually in a localized bond pattern, though the web can be bonded across its entire surface if so desired. Another suitable and well-known bonding method, particularly when using bicomponent staple fibers, is through-air bonding.

Textured coform laminate (TCL): This material was an elastic laminate having outer layers on either side of a core. The outer layers had a basis weight of 35 grams per square meter (gsm) each and made according to the coform process, from a blend of 60 weight percent CF405 fiberized southern softwood pulp from Weyerhaeuser Corp. and 40 weight percent PF-105 polypropylene meltblown fibers from Basell Polyolefins Company N.V. of Hoofddorp, the Netherlands. The core was 30 gsm in basis weight and made of filaments and nonwoven fabric. The filaments comprised 70 weight percent of the core and were made from AFFINITY® metallocene-based polyethylene from the Dow Chemical Company of Midland, MI, USA. The nonwoven fabric was made according to the meltblown process from 80 weight percent AFFINITY® polyethylene, 15 weight percent REGALREZ® 1126 hydrocarbon resin from Eastman Chemical Company of Kingsport, TN, USA and 5 weight percent DNDB 1077 linear low density polyethylene from the Dow Chemical Company.

In the meltblown process, fibers are formed by extruding a molten thermoplastic material through a plurality of fine, usually circular, die capillaries as molten threads or filaments into converging high velocity, usually hot, gas (e.g. air) streams which attenuate the filaments of molten thermoplastic material to reduce their diameter, which may be to microfiber diameter. The meltblown fibers are then carried by the high velocity gas stream and are deposited on a collecting surface to form a web of randomly dispersed meltblown fibers. Such a process is disclosed, for example, in US Patent 3,849,241 to Butin et al. Meltblown fibers are microfibers which may be continuous or discontinuous, are generally smaller than 10 microns in average diameter, and are generally tacky when deposited onto a collecting surface.

In the coform process, at least one meltblown diehead is arranged near a chute through which other materials are added to a meltblown web while it is forming. Such other materials may be natural fibers, superabsorbent particles, natural polymers (for example, rayon) and/or synthetic polymers (for example, polypropylene or polyester) fibers, for example, where the fibers may be of staple length. Coform processes are shown in commonly assigned US Patents 4,818,464 to Lau and 4,100,324 to Anderson et al. Webs produced by the coform process are generally referred to as coform materials. Natural fibers include wool, cotton, flax, hemp and wood pulp. Wood pulps include standard softwood fluffing grade such as CR-1654 (US Alliance Pulp Mills, Coosa, Alabama). Pulp may be modified in order to enhance the inherent characteristics of the fibers and their processability. Curl may be imparted to the fibers by methods including chemical treatment or mechanical twisting. Curl is typically imparted before crosslinking or stiffening. Pulps may be stiffened by the use of crosslinking agents such as formaldehyde or its derivatives, glutaraldehyde, epichlorohydrin, methylolated compounds such as urea or urea derivatives, dialdehydes such as maleic anhydride, non-methylolated urea derivatives, citric acid or other polycarboxylic acids. Some of these agents are less preferable than others due to environmental and health concerns. Pulp may also be stiffened by the use of heat or caustic treatments such as mercerization. Examples of these types of fibers include NHB416 which is a chemically crosslinked southern softwood pulp fibers which enhances wet modulus, available from the Weyerhaeuser Corporation of Tacoma, WA. Other useful pulps are debonded pulp (NF405) and non-debonded pulp (NB416) also from Weyerhaeuser. HPZ3 from Buckeye Technologies, Inc of Memphis, TN, has a chemical treatment that sets in a curl and twist, in addition to imparting added dry and wet stiffness and resilience to the fiber. Another suitable pulp is Buckeye HP2 pulp and still another is IP Supersoft from International Paper Corporation. Suitable rayon fibers are 1.5 denier Merge 18453 fibers from Acordis Cellulose Fibers Incorporated of Axis, Alabama.

HYDROKNIT® material: HYDROKNIT® material is available from Kimberly-Clark Corporation of Dallas, TX, USA and is a hydroentangled web of soft absorbent cellulosic fibers and spunbond synthetic fibers. The synthetic fibers are commonly polypropylene. The materials tested herein had a basis weight of 64 gsm and consists of only one ply of 75 weight percent pulp and 25 weight percent polypropylene spunbond fibers. As used herein the term "HYDROKNIT® with PP fibers" refers to the above described HYDROKNIT® fabric having an additional layer of spunbond polypropylene fibers deposited on its surface. This results in a coarse texture PP fiber layer on the HYDROKNIT® substrate to increase abrasion properties.

The term "spunbonded fibers" refers to small diameter fibers which are formed by extruding molten thermoplastic material as filaments from a plurality of fine, usually circular capillaries of a spinneret with the diameter of the extruded filaments then being rapidly reduced as by, for example, in US Patent 4,340,563 to Appel et al., and US Patent 3,692,618 to Dorschner et al., US Patent 3,802,817 to Matsuki et al., US Patents 3,338,992 and 3,341,394 to Kinney, US Patent 3,502,763 to Hartman, and US Patent 3,542,615 to Dobo et al. Spunbond fibers are generally not tacky when they are deposited onto a collecting surface. Spunbond fibers are generally continuous and have average diameters (from a sample of at least 10) larger than 7 microns, more particularly, between about 10 and 20 microns.

VIVA® Scrub Cloth: This material is a cellulosic paper towel and is available from Kimberly-Clark Corporation. It has a printed polyethylene acetate binder on both sides of the base-sheet which is composed of 72 weight percent softwood bleached kraft, 13 weight percent polyethylene vinyl acetate binder, 11 weight percent synthetic (polyester) fiber, 3 weight percent hardwood kraft, 1 weight percent total nitrogen.

WYPALL® X80 material: WYPALL® materials are also available from Kimberly-Clark Corporation. WYPALL® X80 material is a highly absorbent, bulky HYDROKNIT® material having high wet strength and capacity. The materials tested herein had a basis weight of 125 gsm and were made from 75 weight percent pulp and 25 weight percent polypropylene spunbond fibers.

Airlaid fabric: "Airlaying" is a well-known airforming process by which a fibrous nonwoven layer can be formed. In the airlaying process, bundles of small fibers having typical lengths ranging from about 3 to about 52 millimeters (mm) are separated and entrained in an air supply and then deposited onto a forming screen, usually with the assistance of a vacuum supply. The randomly deposited fibers then are bonded to one another using, for example, hot air or a spray adhesive. The production of airlaid nonwoven composites is well defined in the literature and documented in the art. Examples include the DanWeb process as described in US patent 4,640,810 to Laursen et al. and assigned to Scan Web of North America Inc, the Kroyer process as described in US patent 4,494,278 to Kroyer et al. and US patent 5,527,171 to Soerensen assigned to Niro Separation a/s, the method of US patent 4;375,448 to Appel et al assigned to Kimberly-Clark Corporation, or other similar methods. The materials tested herein were made from 83 weight percent Weyerhaeuser CF405 pulp, and 17 weight percent latex binder (National Starch Dur-O-Set Elite PE) and had a basis weight of 68 gsm.

A number of other processes and materials may be used in the practice of the invention but not all were tested herein. Some of these other materials and processes are described below.

Point un-bonded or "PUB", means a fabric pattern having continuous bonded areas defining a plurality of discrete un-bonded areas as illustrated in US Patent 5,858,515 to Stokes et al. The fibers or filaments within the discrete un-bonded areas are dimensionally stabilized by the continuous bonded areas that encircle or surround each un-bonded area, such that no support or backing layer of film or adhesive is required. The un-bonded areas are specifically designed to afford spaces between fibers or filaments within the un-bonded areas. A suitable process for forming point un-bonded nonwoven material includes providing a nonwoven fabric or web, providing opposedly positioned first and second calender rolls and defining a nip therebetween, with at least one of the rolls being heated and having a bonding pattern on its outermost surface comprising a continuous pattern of land areas defining a plurality of discrete openings, apertures or holes, and passing the nonwoven fabric or web within the nip formed by the rolls. Each of the openings in the roll or rolls defined by the continuous land areas forms a discrete un-bonded area in at least one surface of the nonwoven fabric or web in which the fibers or filaments of the web are substantially or completely un-bonded. Stated alternatively, the continuous pattern of land areas in the roll or rolls forms a continuous pattern of bonded areas that define a plurality of discrete un-bonded areas on at least one surface of the nonwoven fabric or web. Alternative embodiments of the aforesaid process includes pre-bonding the nonwoven fabric or web before passing the fabric or web within the nip formed by the calender rolls, or providing multiple nonwoven webs to form a pattern-un-bonded laminate.

The zero strain stretch process generally refers to a process in which at least two layers are bonded to one another while in an untensioned (hence zero strain) condition and where one of the layers is stretchable and elastomeric and the second is stretchable but not necessarily elastomeric. Such a laminate is stretched incrementally through the use of one or more pairs of meshing corrugated rolls which reduce the strain rate experienced by the web. This results in z-direction bulking of the laminate and subsequent elastic extensibility in the direction of initial stretching at least up to the point of initial stretching. Examples of such laminates and their production processes may be found in US Patents 5,143,679, 5,151,092, 5,167,897, and 5,196,000.

Z - directionally oriented fiber webs may also be used in the practice of this invention. A discussion of this process may be found in the October 1997 issue of Nonwovens Industry magazine at page 74 in an article by Krema, Jirsak, Hanus and Saunders entitled "What's New in Highloft Production?" as well as in Czech patents 235494 entitled "Fibre Layer, Method of its Production and Equipment for Application of Fibre Layer Production Method" issued May 15, 1995 and 263075 entitled "Method for Voluminous Bonded Textiles Production" issued April 14, 1989.

Another suitable material includes those made according to US patent 4,741,941, which teaches a nonwoven web with projections. The web from which the product will be made is formed onto a surface having projections with or without apertures and having a vacuum assist. The fabric has fibers with an array of hollow projections extending out of the fabric and separated by planar land areas. Fabric of this type may be made according to any of the nonwoven production techniques such as meltblowing, spunbonding, airlaying and the like.

Suitable products also include those taught in US patents 4,775,582, 4,853,281 and 4,833,003. The '582 and '281 patents teach uniformly moist wipes made from polyolefin meltblown fibers. The '003 patent teaches uniformly moist wipes that have an abrasive surface bonded to a meltblown supporting layer.

A number of different treatments were used in the Experiments described below. The treatment of the webs was done as follows:
KYMENE® 2064: A 0.1 weight percent KYMENE® 2064 solution was prepared by diluting a stock KYMENE® 2064 (from Hercules Inc., Wilmington, DE, USA) solution (20 weight percent solution in water, 5 mL) with de-ionized water (995 mL). KYMENE® 2064 was "activated" by adjusting the solution pH with NaOH (0.4 M), which was measured at 8.8. Treatment of the substrates entailed a "dip and squeeze" protocol. Each substrate was submerged in the 0.1 weight percent KYMENE® 2064 solution and agitated for approximately 1 minute to ensure saturation. The treated material was then squeezed to remove excess treatment solution using an Atlas Laboratory Wringer Type LW-1 (Atlas Electrical Devices Co., Chicago, IL, USA) equipped with a '2268g (5 lb) weight for the squeeze pressure. The material was cured at 100 °C for 20 minutes, allowed to cool to room temperature, and washed twice with de-ionized water. Excess water was removed using the same "dip and squeeze" protocol above. The washed material was allowed to dry at 100 °C for 30 minutes.
KYMENE® 450: A 0.1 weight percent KYMENE® 450 solution was prepared by diluting a stock KYMENE® 450 (Hercules Inc.) solution (20 weight percent solution in water, 5 mL) with de-ionized water (995 mL). KYMENE® 450 was "activated" by adjusting the solution pH with NaOH (0.4 M), which was measured at 9.2. Treatment of the substrates was performed in the same manner as with KYMENE® 2064 above.
KYMENE® 557 LX: A 0.1 weight percent KYMENE® 557 LX solution was prepared by diluting a stock KYMENE® 557 LX (Hercules Inc.) solution (12.5 weight percent solution in water, 8 mL) with de-ionized water (992 mL). The solution pH was adjusted with NaOH (0.4 M), which was measured at 8.0. Treatment of the substrates was performed in the same manner as with KYMENE® 2064 above.
KYMENE® 736: A 0.1 weight percent KYMENE® 736 solution was prepared by diluting a stock KYMENE® 736 (Hercules Inc., Wilmington, DE) solution (38 weight percent solution in water, 2.6 mL) with de-ionized water (997.4 mL). The solution pH was adjusted with NaOH (0.4 M), which was measured at 8.0. Treatment of the substrates was performed in the same manner as with KYMENE® 2064 above.
Alumina oligomer (aluminum chlorohydrol, aluminum chlorohydrate): A 1 weight percent alumina oligomer solution was prepared by diluting a stock alumina oligomer (from GEO Specialty Chemicals, Little Rock, AR, USA) solution (50 weight percent solution in water, 20 mL) with de-ionized water (980 mL). The measured pH was 4.6. Treatment of the substrates entailed a "dip and squeeze" protocol. Each substrate was submerged in the 1 weight percent alumina oligomer solution and agitated for approximately 1 min to ensure saturation. The treated material was then squeezed to remove excess treatment solution using an Atlas Laboratory Wringer Type LW-1 (Atlas Electrical Devices Co.) equipped with a '2268g (5 lb) weight for the squeeze pressure. The material was heated at 100 °C for 20 minutes, allowed to cool to room temperature, and washed twice with de-ionized water. Excess water was removed using the same "dip and squeeze" protocol above. The material was allowed to dry at 100 °C for 30 minutes.
SNOWTEX® AK nanoparticle (alumina-coated silica nanoparticles): A 1 weight percent SNOWTEX® AK nanoparticle solution was prepared by diluting a stock SNOWTEX® AK nanoparticle (from Nissan Chemicals Ltd, Houston, TX, USA) solution (20 weight percent solution in water, 75 mL) with de-ionized water (1425 mL). The measured pH was 4.0. Treatment of the substrates entailed a "dip and squeeze" protocol. Each substrate was submerged in 1 weight percent SNOWTEX® AK nanoparticle solution and agitated for approximately 1 min to ensure saturation. The treatment solution for each substrate was not recycled for subsequent treatments. The treated material was then squeezed to remove excess treatment solution using an Atlas Laboratory Wringer Type LW-1 (Atlas Electrical Devices Co.) equipped with a 5 lb weight for the squeeze pressure. The material was heated at 100 °C for 20 minutes, allowed to cool to room temperature, and washed twice with de-ionized water. Excess water was removed using the same "dip and squeeze" protocol above. The material was allowed to dry at 100 °C for 30 minutes.

### Experiment 1- bacterial growth inhibition test (Reference)

The KYMENE® class of chemicals are generally mild and not caustic to the skin. Certain of the KYMENE® chemicals, however, are known to kill bacteria at some level. In order to determine whether the KYMENE® treatment chemicals will escape from a substrate and perhaps kill the bacteria remaining on a surface, an assay was designed to measure the inhibition in bacterial cell growth of chemical leached from the treated materials. This assay procedure follows.

Two by two inch (5 by 5 cm) squares of treated and untreated materials were placed in 15 mL tubes containing 5 mL of sterile phosphate-buffered saline (PBS) solution. The tubes were placed in a shaking incubator at 37 °C for 2-3 hours. One milliliter of each solution was then transferred into a clean culture tube. Ampicillin-resistant *E. Coli* was added (10 microL, ∼1000 cells) to each tube. Sterile PBS was added to clean culture tubes as controls. The tubes were returned to the shaking incubator for 30 minutes more. One hundred microliters were removed from each tube and plated onto LB agar plates containing ampicillin. The plates were incubated at 37 °C, and bacterial colonies were counted the following day to determine if there was KYMENE® present in the solution that inhibited colony formation.

LB agar means Luria-Bertani broth (available from Difco and Becton Dickinson) in the amount of 25 grams mixed with agar (also from Difco and Becton Dickinson) in the amount of 15 grams and dissolved in 1 liter of distilled water and autoclaved. Circular plates (100 mm x 15 mm) are poured after adding ampicillin (100 micrograms/mL) to the LB agar.

Data are listed in terms of the percent of colonies found on the plate compared to the PBS control.

| **Materials** | **Type of KYMENE®** | **Percent of Control** |
|---|---|---|
| PBS Control | - | 100 |
| Spunlace | - | 100 |
| Spunlace | 2064 | 75 |
| Spunlace, softened | - | 87 |
| Spunlace, softened | 2064 | 82 |
| Fuzzy film, polyurethane | - | 84 |
| Fuzzy film, polyurethane | 2064 | 75 |
| Bonded Carded Web | - | 81 |
| Bonded Carded Web | 2064 | 100 |
| TCL | - | 80 |
| TCL | 2064 | 81 |
| TCL | 736 | 3 |
| HYDROKNIT® | - | 93 |
| HYDROKNIT® | 2064 | 81 |
| HYDROKNIT® with PP fibers | - | 69 |
| HYDROKNIT® with PP fibers | 2064 | 38 |
| VIVA® Scrub Cloth | - | 100 |
| VIVA® Scrub Cloth | 2064 | 95 |
| WYPALL® X80 | - | 100 |
| WYPALL® X80 | Aegis quaternary ammonium salt | 100 |
| WYPALL® X80 | 2064 | 87 |
| WYPALL® X80 | 736 | 14 |

As can be seen from the results, none of the untreated materials except for the HYDROKNIT® with polypropylene (PP) fibers appeared to have had a dramatic effect on colony growth. The KYMENE® 2064 materials did not show appreciable inhibition of growth except for the HYDROKNIT® with PP fibers. Materials treated with KYMENE® 736 leached KYMENE® into the solution, which killed most of the E. *Coli* in solution. This result is not surprising since it is known that KYMENE® 2064 will cross-link to the above listed materials while KYMENE® 736 generally will not. Treatments that are cross-linked to the substrate are more stable and less susceptible to leaching than are un-cross-linked treatments and are therefore desirable. A successful treatment will have at least 80 percent of the control sample cell colony growth, i.e., it will inhibit growth of 20 percent or less of the bacterial colonies.

### Experiment 2- bacterial growth inhibition test

SNOWTEX® nanoparticles and aluminum oligomer were tested directly on E. *Coli.* Serial dilutions of both SNOWTEX® nanoparticles as well as the aluminum oligomer used to coat the nanoparticles were made in sterile PBS. One milliliter of each solution was added to a clean culture tube in duplicate. Sterile PBS was added to culture tubes as a control. Ampicillin-resistant E. *Coli* was added (10 microL, ∼1000 cells) to each solution. The culture tubes were placed in the 37 °C shaking incubator for 30 minutes. After the incubation, one hundred microliters were removed from each tube and plated onto LB agar plates containing ampicillin. Plates were incubated at 37 °C, and bacterial colonies were counted the following day to determine if SNOWTEX® nanoparticles or the aluminum oligomer inhibited colony formation. Data are listed in terms of the percent of colonies found on the plate compared to the PBS control.

| **SNOWTEX® AK nanoparticle** | **Percent of Control** | **Al Oligomer** | **Percent of Control** |
|---|---|---|---|
| PBS control | 100 | PBS Control | 100 |
| 10 mg/mL | 96 | 1% | 10 |
| 1 mg/mL | 99 | 0.5% | 57 |
| 0.5 mg/mL | 94 | 0.1% | 100 |
| 0.1 mg/mL | 100 | 0.05% | 100 |
| 0.05 mg/mL | 93 | 0.01% | 100 |
| 0.01 mg/mL | 89 | - | - |

The results show that SNOWTEX® nanoparticle did not have an effect on *E. Coli* colony formation, even at high concentrations (10 mg/mL). The aluminum oligomer decreased the number of *E. Coli* colonies when at concentrations 0.5% and higher. The concentration of the aluminum oligomer used to treat the nanoparticles is 1%. These results indicate that bacteria cell death will be observed only if all of the oligomer used to treat the materials leaches into the solution. As mentioned above, a successful treatment will have at least 80 percent survival of the control sample colonies, i.e., it will kill 20 percent or less of the bacterial colonies.

### Experiment 3-Method of testing the efficiency of binding bacteria:

Not only must the successful treatment not kill substantial numbers of bacteria, it must also bind a large proportion of bacteria. In order to determine how efficient the substrate and treatment were in holding bacteria cleaned from the surface, the following test procedure was carried out.

Two by two inch (5 by 5 cm) squares of materials were cut and weighed in duplicate. Serial dilutions of an ampicillin-resistant *E*. *Coli* solution were made to achieve a final concentration of ∼10⁵ cells per mL. One hundred microliters of sterile PBS were added to each material. After 5 minutes, one hundred microliters of the bacteria solution were added onto each material. The materials were removed and placed into 10 mL of sterile PBS in 50 mL tubes. The tubes were sonicated (5 cycles of 30 seconds on, 30 seconds off) in a water bath to dislodge any bacteria that is not bound tightly to the material. One hundred microliters of the PBS solution from the tubes containing the material were plated in duplicate onto LB agar plates containing ampicillin. The plates were incubated at 37 °C and bacterial colonies were counted the following day. Data is shown as the percentage of reduction of bacteria in solution as compared to the PBS control.

| **Material** | | **Treatment** | **Reduction of Bacteria in Solution (%) N=4** |
|---|---|---|---|
| PBS control ** | | - | 0 |
| WYPALL® X80 ** | | - | 62 |
| WYPALL®X80 ** | | KYMENE® 2064 | 72 |
| WYPALL®X80 ** | | KYMENE® 450 | 63 |
| WYPALL® X80 ** | | KYMENE@ 557 | 40 |
| WYPALL® X80 | | Al oligomer | 84 |
| WYPALL® X80 | | SNOWTEX® AK nanoparticle | 84 |
| TCL ** | | - | 47 |
| TCL ** | | KYMENE® 2064 | 77 |
| TCL ** | | KYMENE® 450 | 70 |
| TCL ** | | KYMENE® 557 | 41 |
| TCL* | | Al oligomer | 94,97 |
| TCL | | SNOWTEX® AK nanoparticle | 95 |
| VIVA® Scrub Cloth* ** | | - | 58, 48 |
| VIVA® Scrub Cloth* ** | | KYMENE® 2064 | 83, 73, 78 |
| VIVA® Scrub Cloth ** | | KYMENE® 450 | 59 |
| VIVA® Scrub Cloth ** | | KYMENE® 557 | 80 |
| VIVA® Scrub Cloth | | Al oligomer | 82 |
| VIVA® Scrub Cloth | | SNOWTEX® AK nanoparticle | 68 |
| | Airlaid ** | - | 10 |
| | Airlaid ** | KYMENE® 2064 | 64 |
| | Airlaid ** | KYMENE@ 450 | 67 |
| | Airlaid ** | KYMENE® 557 | 36 |
| | Airlaid | Al oligomer | 75 |
| | Airlaid | SNOWTEX® AK nanoparticle | 57 |

| | | | |
|---|---|---|---|
| * Materials tested multiple times ** Comparative | | | |

All materials, treated and untreated, showed a reduction in bacteria in the PBS solution after sonication. The most dramatic results are found in materials treated with KYMENE® 2064, KYMENE® 450, the aluminum oligomer, and in some cases, the SNOWTEX® AK nanoparticle nanoparticles. In all cases except for the WYPALL® material, the treated materials showed a larger reduction in bacteria in solution than the untreated materials. It is desirable that the treated materials reduce bacterial growth according to this bacteria binding procedure by at least 50 percent, more desirably by at least 75 percent and still more desirably by at least 90 percent.

### Experiment 4- Streaming Zeta Potential Analysis- Used to measure the surface charge of treated substrates:

When an electrolyte solution is forced through a porous plug of material, a streaming potential develops due to the motion of ions in the diffusion layer which can be measured by an Electro Kinetic Analyzer (from Brookhaven Instruments Corporation, Holtsville, NY, USA). This value is then used to calculate the zeta potential according to the formula published by D. Fairhurst and V. Ribitsch (Particle Size Distribution II, Assessment and Characterization, Chapter 22, ACS Symposium Series 472, Edited by Provder, Theodore, ISBN 0841221170).

During the sample preparation, treated and untreated substrates were cut to two identical pieces (120 mm x 50 mm) and then placed into the sample cell with TEFLON® spacers between them. After the sample cell was mounted onto the instrument, all the air bubbles were removed by purging. Then KCI solution (1 mM, pH = 5.9, Temp = 22 °C) was forced through the two layers of the media and Ag/AgCl electrodes were used to measure the streaming potential. All samples were tested under similar pH, solution conductivity and using the same number of spacers.

Each testing was repeated 4 times, and the results are summarized in the table below.

| **Material** | | **Treatment** | **Streaming Zeta Potential (mV)** |
|---|---|---|---|
| WYPALL® X80 ** | | - | -1 |
| WYPALL® X80 ** | | KYMENE® 2064 | +11 |
| WYPALL® X80 ** | | KYMENE® 450 | +12 |
| WYPALL® X80 ** | | KYMENE® 557 | +5 |
| WYPALL® X80 | | Al oligomer | +8 |
| WYPALL® X80 | | SNOWTEX® AK | +25 |
| | TCL ** | - | -2 |
| | TCL ** | KYMENE® 2064 | +29 |
| | TCL ** | KYMENE® 450 | +33 |
| | TCL ** | KYMENE® 557 | +15 |
| | TCL* | Al oligomer | +25 |
| | TCL | SNOWTEX® AK | +27 |
| VIVA® Scrub Cloth* ** | | - | -11 |
| VIVA® Scrub Cloth* ** | | KYMENE@ 2064 | +23 |
| VIVA® Scrub Cloth ** | | KYMENE® 450 | +22 |
| VIVA® Scrub Cloth** | | KYMENE® 557 | +11 |
| VIVA® Scrub Cloth | | Al oligomer | +7.3 |
| VIVA® Scrub Cloth | | SNOWTEX® AK | +13 |
| | Airlaid** | - | -6 |
| | Airlaid** | KYMENE® 2064 | +40 |
| | Airlaid** | KYMENE® 450 | +38 |
| | Airlaid** | KYMENE@ 557 | +31 |
| | Airlaid | Al oligomer | +17 |
| | Airlaid | SNOWTEX® AK | +25 |

| | | | |
|---|---|---|---|
| ** Comparative | | | |

As can be seen from the data, the zeta potential for untreated substrates was negative, ranging from -11 mV to - 1 mV at pH - 5.9. The negative values for the untreated substrates indicate there should be repulsion between most bacteria and the untreated substrates. After treatment, the zeta potential for all the substrates became positive. The most cationically charged substrates are found to be materials treated with KYMENE® 2064, KYMENE® 450, the aluminum oligomer, and SNOWTEX® AK nanoparticles.

### Experiment 5- Removal of bacteria through wiping:

Aluminum chlorohydrate treated (1 weight percent) and untreated TCL materials were cut into 5 cm x 15 cm samples. The materials were soaked in sterile filtered PBS (3 mL per sample) for two hours before wiping experiments commenced. Serial dilutions of an ampicillin-resistant *E. Coli* solution were made to achieve a final concentration of ∼10⁶ cells per mL. Five hundred microliters of the 10⁶ cells per mL *E. Coli* cell solution were spotted onto a piece of ceramic tile. The material sample was placed on top of the *E*. *Coli* spot, and a number of wipes (1-5) were performed. After wiping, the entire run of the tile surface was swabbed for bacteria. The swabs were placed into 1 mL of sterile PBS. One hundred microliters of the PBS solution were plated in duplicate onto ampicillin-containing LB agar plates. Data are described as the percent of residual bacteria found on the tile after wiping and are presented in the table below.

| Material | Input Bacteria | Number of Wipes | Residual Colonies (x 0.1) | % Removal of Bacteria |
|---|---|---|---|---|
| TCL | 7.5 x 10⁵ | 1 | 211 | 99.7 |
| | | 2 | 75 | 99.9 |
| | | 3 | 167 | 99.8 |
| | | 4 | 28 | 99.96 |
| | | 5 | 46 | 99.94 |
| | 9.35 x 10⁵ | 1 | 328 | 99.6 |
| | | 2 | 173 | 99.8 |
| | | 3 | 336 | 99.6 |
| | | 4 | 295 | 99.7 |
| | | 5 | 53 | 99.9 |
| | 8.45 x 10⁵ | 1 | 123 | 99.9 |
| | | 2 | 45 | 99.95 |
| | | 3 | 19 | 99.98 |
| | | 4 | 18 | 99.98 |
| | | 5 | 290 | 99.7 |
| Aluminum Chlorohydrate TCL | 7.5 x 10⁵ | 1 | 98 | 99.9 |
| | | 2 | 46 | 99.94 |
| | | 3 | 1 | 99.999 |
| | | 4 | 2 | 99.997 |
| | | 5 | 1 | 99.999 |
| | 9.35 x 10⁵ | 1 | 88 | 99.9 |
| | | 2 | 77 | 99.9 |
| | | 3 | 39 | 99.96 |
| | | 4 | 6 | 99.99 |
| | | 5 | 10 | 99.99 |
| | 8.45 x 10⁵ | 1 | 26 | 99.97 |
| | | 2 | 25 | 99.97 |
| | | 3 | 24 | 99.97 |
| | | 4 | 8 | 99.99 |
| | | 5 | 8 | 99.99 |

### Experiment 6- Transfer of bacteria through wiping:

Wiping was performed using treated and untreated TCL materials as described in Experiment 5 above. After four wipes, a sterile tile surface was wiped four times with the material containing the bacteria. The tile surface was swabbed as described above to capture any bacteria that was transferred onto the surface. The swabs were placed in 1 mL sterile PBS. One hundred microliters of the PBS solution were plated in duplicate onto ampicillin-containing LB agar plates. The data are presented in the table below (data are number of colonies found on LB/Ampicillin plates).

| Material | Residual Bacteria | Transferred Bacteria |
|---|---|---|
| TCL | 52 | 4 |
| | 15 | 59 |
| | 165 | 6 |
| AI-TCL | 1 | 0 |
| | 4 | 1 |
| | 5 | 1 |

| | | |
|---|---|---|
| Input bacteria = 7.1 x 10⁵ cells | | |

| Material | Transferred Bacteria |
|---|---|
| TCL | 68 |
| | 104 |
| | 80 |
| AI-TCL | 3 |
| | 1 |
| | 0 |

| | |
|---|---|
| Input bacteria = 7.0 x 10⁵ cells | |

### Experiment 7- Transfer of bacteria through direct contact:

Another set of experiments was performed in which the bacteria were added directly to the test material that rested on a ceramic tile. Aluminum chlorohydrate (1 weight percent) treated TCL, Scott® paper towels, and sponges (ScotchBrite® from 3M) were placed on a ceramic tile. Five hundred microliters of a bacteria-containing solution (∼5 x 10⁵ cells) were added directly to each material. The material was lifted off of the tile, and any bacteria that had leaked through the material was removed with a swab that was placed in 1 mL of PBS. The material was then placed bacteria-side down onto a clean, sterile tile. A 1.2 kg weight was then placed on top of the material for a few minutes. The weight and material were removed, and any bacteria that were transferred onto the clean tile surface were removed with a swab that was placed in 1 mL of PBS. One hundred microliters of the PBS/swab solutions were plated in duplicate. The data are presented in the table below (data are number of colonies found on LB/Ampicillin plates.)

| Material | Bacteria Breakthrough | Transferred Bacteria |
|---|---|---|
| AI-TCL | 0 | 0 |
| | 0 | 1 |
| | 0 | 0 |
| Scott® Paper Towel | >500 | 44 |
| | >500 | 223 |
| | >500 | 151 |
| ScotchBrite® Sponge | 0 | 24 |
| (15 washes) | 0 | 19 |
| | 0 | 1 |

| | | |
|---|---|---|
| Input bacteria = 5.8 x 10⁵ cells | | |

| Material | Transferred Bacteria |
|---|---|
| AI-TCL | 2 |
| | 2 |
| | 4 |
| Sponge | 19 |
| (20 washes) | 13 |
| | 27 |
| Scott® Paper Towel | 199 |

### Experiment 8- Visual indication of capture:

Another set of experiments was performed in which blue colored, negatively charged microbeads of a size similar to bacteria (about 1µm) were used to provide visual evidence of capture. Microbeads were diluted 1:3 in PBS. One hundred microliters of the microbead solution were added to a baby wipe untreated TCL and TCL treated as described above with aluminum chlorohydrate. The substrates were then washed vigorously in deionized water. After washing, the untreated substrates were substantially free of blue coloring, showing the microbeads had washed off. The treated TCL clearly retained the blue color, showing the retention of the microbeads.

As will be appreciated by those skilled in the art, changes and variations to the invention are considered to be within the ability of those skilled in the art. Examples of such changes are contained in the patents identified above. Such changes and variations are intended by the inventors to be within the scope of the invention. It is also to be understood that the scope of the present invention is not to be interpreted as limited to the specific embodiments disclosed herein, but only in accordance with the appended claims when read in light of the foregoing disclosure.

## Claims

1. A wipe for the removal of negatively charged particles comprising a substrate having thereon a positively charged chemical compound selected from positively charged nanoparticles and an alumina oligomer, wherein said chemical compound allows at least 80 percent survival of a bacterial colony, and wherein said substrate is made from a nonwoven fabric made according to a method selected from the group consisting of meltblowing, coforming, spunbonding, airlaying, bonding and carding, zero strain stretching and Z-directional orienting.

2. The wipe of claim 1, where the positively charged chemical compound is embedded in melt-extruded polymer fibers.

3. The wipe of claim 1, where the positively charged chemical compound is applied to a surface of said substrate.

4. The wipe of claim 3, where said substrate is treated with heat at a temperature and for a time sufficient to adhere said compound to said substrate.

5. The wipe of claim 3, wherein said chemical compound is applied to said substrate in an amount between 0.01 and 10 weight percent on an aqueous basis.

6. The wipe of claim 3, wherein said chemical compound is applied to said substrate in an amount between 0.05 and 7 weight percent on an aqueous basis.

7. The wipe of claim 3, wherein said chemical compound is applied to said substrate in an amount between 0.1 and 5 weight percent on an aqueous basis.

8. The wipe of claim 1, wherein said substrate reduces bacterial growth according to a bacteria binding procedure by at least 50 percent.

9. The wipe of claim 1, wherein said substrate reduces bacterial growth according to a bacteria binding procedure by at least 75 percent.

10. The wipe of claim 1, wherein said substrate reduces bacterial growth according to a bacteria binding procedure by at least 90 percent.

11. The wipe of claim 4 comprising hydroentangled pulp and synthetic fibers.

12. The wipe of claim 1, for the removal of bacteria from surfaces comprising a web of pulp and synthetic fibers having thereon a cationic nanoparticle treatment.

## Patentansprüche

1. Wischtuch zum Entfernen von negativ geladenen Partikeln, umfassend ein Substrat mit einer positiv geladenen chemischen Verbindung darauf, ausgewählt aus positiv geladenen Nanopartikeln und einem Aluminiumoxid-Oligomer, wobei die chemische Verbindung mindestens 80 Prozent Überleben einer Bakterienkolonie zulässt, und wobei das Substrat aus einem Vlies hergestellt ist, welches hergestellt ist gemäß einem Verfahren, ausgewählt aus der Gruppe, bestehend aus Schmelzblasen, Coformen, Spunbonding, Luftablegen, Bonden und Kardieren, Spannungsloses Strecken und Orientieren in Z-Richtung.

2. Wischtuch nach Anspruch 1, wobei die positiv geladene chemische Verbindung in schmelzextrudierten Polymerfasern eingebettet ist.

3. Wischtuch nach Anspruch 1, wobei die positiv geladene chemische Verbindung auf eine Oberfläche des Substrats aufgetragen ist.

4. Wischtuch nach Anspruch 3, wobei das Substrat mit Wärme bei einer Temperatur und während eines Zeitraums behandelt ist, welche ausreichend sind um die Verbindung an das Substrat anzuhaften.

5. Wischtuch nach Anspruch 3, wobei die chemische Verbindung in einer Menge zwischen 0,01 und 10 Gewichtsprozent auf einer wässrigen Basis auf das Substrat aufgetragen ist.

6. Wischtuch nach Anspruch 3, wobei die chemische Verbindung in einer Menge zwischen 0,05 und 7 Gewichtsprozent auf einer wässrigen Basis auf das Substrat aufgetragen ist.

7. Wischtuch nach Anspruch 3, wobei die chemische Verbindung in einer Menge zwischen 0,1 und 5 Gewichtsprozent auf einer wässrigen Basis auf das Substrat aufgetragen ist.

8. Wischtuch nach Anspruch 1, wobei das Substrat gemäß einer Bakterienbindungsmethode Bakterienwachstum um mindestens 50 Prozent verringert.

9. Wischtuch nach Anspruch 1, wobei das Substrat gemäß einer Bakterienbindungsmethode Bakterienwachstum um mindestens 75 Prozent verringert.

10. Wischtuch nach Anspruch 1, wobei das Substrat gemäß einer Bakterienbindungsmethode Bakterienwachstum um mindestens 90 Prozent verringert.

11. Wischtuch nach Anspruch 4, umfassend wasserstrahlverfestigte Zellstoff- und Synthesefasern.

12. Wischtuch nach Anspruch 1, zum Entfernen von Bakterien von Oberflächen, umfassend eine Bahn von Zellstoff- und Synthesefasern mit einer kationischen Nanopartikel-Behandlung darauf.

## Revendications

1. Lingette pour l'élimination de particules à charge négative, comprenant un substrat sur lequel se trouve un composé chimique à charge positive choisi parmi les nanoparticules à charge positive et un oligomère d'alumine, ledit composé chimique permettant un taux de survie d'au moins 80 pour cent pour une colonie bactérienne et ledit substrat étant composé d'un papier non tissé préparé selon un procédé choisi dans le groupe constitué d'un procédé de fusion-soufflage, d'un procédé de coformation, d'un procédé de filage-nappage, d'un procédé de formation par voie hydrodynamique, d'un procédé de liaison et de cardage, d'un procédé d'étirage à tension nulle et d'un procédé d'orientation dans la direction de Z.

2. Lingette selon la revendication 1, où le composé chimique à charge positive est enrobé dans des fibres polymères extrudées à l'état fondu.

3. Lingette selon la revendication 1, où le composé chimique à charge positive est appliqué sur une surface dudit substrat.

4. Lingette selon la revendication 3, où ledit substrat est soumis à un traitement thermique à une température suffisante et pendant une durée suffisante pour que ledit composé adhère audit substrat.

5. Lingette selon la revendication 3, dans laquelle ledit composé chimique est appliqué sur ledit substrat en une quantité entre 0,01 et 10 pour cent en poids sur une base aqueuse.

6. Lingette selon la revendication 3, dans laquelle ledit composé chimique est appliqué sur ledit substrat en une quantité entre 0,05 et 7 pour cent en poids sur une base aqueuse.

7. Lingette selon la revendication 3, dans laquelle ledit composé chimique est appliqué sur ledit substrat en une quantité entre 0,1 et 5 pour cent en poids sur une base aqueuse.

8. Lingette selon la revendication 1, dans laquelle ledit substrat réduit la croissance bactérienne selon une procédure de liaison de bactéries d'au moins 50 pour cent.

9. Lingette selon la revendication 1, dans laquelle ledit substrat réduit la croissance bactérienne selon une procédure de liaison de bactéries d'au moins 75 pour cent.

10. Lingette selon la revendication 1, dans laquelle ledit substrat réduit la croissance bactérienne selon une procédure de liaison de bactéries d'au moins 90 pour cent.

11. Lingette selon la revendication 4, comprenant des fibres de pâte et synthétiques hydroenchevêtrées.

12. Lingette selon la revendication 1, pour éliminer les bactéries de surfaces, comprenant une nappe de pâte et de fibres synthétiques sur lesquelles se trouve un traitement à base de nanoparticules cationiques.
